# EUROPEAN PATENT SPECIFICATION

(11) **EP 3 710 828 B1**
(45) Date of publication and mention of the grant of the patent: **07.06.2023**
(21) Application number: 18800161.4
(22) Date of filing: 09.11.2018
(51) Int. Cl.: G01N 30/88, D06H 3/00, G01N 33/36, G01N 1/40

(54) **METHOD OF DEMONSTRATING SEBUM REMOVAL FROM LAUNDERED GARMENTS**
VERFAHREN ZUR DEMONSTRATION DER SEBUMENTFERNUNG AUS GEWASCHENEN KLEIDUNGSSTÜCKEN
PROCÉDÉ DE DÉMONSTRATION DE L'ÉLIMINATION DE SÉBUM DE VÊTEMENTS BLANCHIS

(30) Priority: 13.11.2017 EP 17201436
(43) Date of publication of application: 23.09.2020
(73) Proprietor: Unilever IP Holdings B.V., 3013 AL Rotterdam (NL); Unilever Global IP Limited, Wirral, Merseyside CH62 4ZD (GB)
(72) Inventor: BATCHELOR, Stephen, Norman, Bebington Wirral Merseyside CH63 3JW (GB); BURNHAM, Neil, Stephen, Bebington Wirral Merseyside CH63 3JW (GB); SANDERSON, Alastair, Richard, Bebington Wirral Merseyside CH63 3JW (GB)
(74) Representative: McHugh, Paul Edward
(86) International application number: PCT/EP2018/080789
(87) International publication number: WO 2019/092191

(56) References cited:
- EP-A1- 2 535 401
- WO-A1-2006/092689
- US-A- 6 090 768
- SIGNE MUNK ET AL: "Primary odorants of laundry soiled with sweat/sebum: Influence of lipase on the odor profile", JOURNAL OF SURFACTANTS AND DETERGENTS, vol. 3, no. 4, 1 October 2000 (2000-10-01) , pages 505-515, XP055328963, DE ISSN: 1097-3958, DOI: 10.1007/s11743-000-0150-z
- KOHEI TAKEUCHI ET AL: "Identification of novel malodour compounds in laundry", FLAVOUR AND FRAGRANCE JOURNAL, vol. 27, no. 1, 22 September 2011 (2011-09-22), pages 89-94, XP055107825, ISSN: 0882-5734, DOI: 10.1002/ffj.2088
- MATA A PAULINA DE LA ET AL: "Comprehensive two-dimensional gas chromatographic profiling and chemometric interpretation of the volatile profiles of sweat in knit fabrics", ANALYTICAL AND BIOANALYTICAL CHEMISTRY, SPRINGER, DE, vol. 409, no. 7, 27 December 2016 (2016-12-27), pages 1905-1913, XP036155841, ISSN: 1618-2642, DOI: 10.1007/S00216-016-0137-1 [retrieved on 2016-12-27]

## Description

### Field of Invention

The present invention concerns a method of demonstrating sebum removal from laundered clothes by laundry detergent compositions.

### Background of the Invention

Clothing may contain a range of organic staining materials that are invisible to the human eye, these invisible stains include food components, pollutants and human sebum. Assessment of the efficacy of a laundry product at removal of the various invisible stains is a challenging task, due to the complex nature of the stain.

Human sebum is a complex mixture of components which include a wide variety of free fatty acids, di and triglycerides, wax esters, cholesterol esters and squalene. Many of the components are unique to Homo Sapiens, for example sapienic acid the major fatty acid in sebum is unique to humans. When garments are worn next to the skin, a large amount of sebum is transferred to the garment. The amount transferred depends upon the garment type, weather and the person wearing the garment. Removal of this sebum is a main function of laundry detergent products. In the early stages residual sebum is invisible to the naked eye, over multiple wash-wear cycles it leads to garment yellowing and odour over time.

Bey in Fette, Seifen, Anstrichmittel, 1963, vol 65, p611 analysed the composition of sebum from worn clothes using by solvent extraction of worn shirts, then analysis via chromatographic separation of the components -in combination with IR spectroscopy, Gas-chromatography with a flame ionisation detector and comparison to standards.

Powe (Powe, W.C. & Marple, W.L. J Am Oil Chem Soc (1960) 37: 136.), analysed the fatty acid composition of sebum from worn clothing using a soxhlet extraction with various organic solvents, then alkaline hydrolysis of the glycerides followed by esterification and gas chromatographic analysis with comparison to known standards. It is unclear in this procedure if wax esters and cholesterol esters were also extracted and included in the fatty acid analysis.

Signe Munk et al (Journal of Surfactants and Detergents, vol. 3, no. 4, 1 October 2000) has measured the odorants of sweat/sebum after laundering using gas chromatography and mass spectroscopy with electron impact or chemical ionization. This document detects the volatile compounds and the method cannot detect the majority of the compounds in sebum which are non-volatile. This is illustrated by the results of D1 where a lipase shows little effects on the results. This document is not an effective method to measure the majority of sebum components.

A simpler method is required that allows the chemical identification and quantification of the sebum stain components on clothes, and further for the comparison of laundry product efficacy at sebum removal from clothes.

### Summary of the Invention

The invention relates to a method of demonstrating sebum removal from laundered garment(s) by one or more laundry detergent compositions, said method comprising:
a) human subject to wear garment next to skin, preferably for a time of from 5 to 100 hours, more preferably the garment is worn on the upper half of the body;
b) the worn garment is portioned into 2 or more equal portions;
c) garment portions are washed separately to each other using different laundry detergent compositions to enable a comparison to be made;
d) The garment portions are dried and stored, preferably stored for a maximum of 96 hours at a temperature below 293K;
e) each garment portion or equal part thereof undergoes solvent extraction with solvent selected from: alcohols, ketones, ethers, CO₂, chlorinated organic solvent, preferably the solvent is acetone or chloroform;
f) take the solvent extract and choose method selected from:
   (i) use as is; remove all solvent and then redissolve in a solvent mixture, preferably selected from an ether/alcohol mixture, chlorinated organic solvent /alcohol, or chlorinated organic solvent /ether/alcohol mixture, most preferably the ether is tert-butyl methyl ether, the chlorinated organic solvent is chloroform and the alcohol selected from methanol, ethanol and propan-2-ol; or
   (ii) reduce solvent volume to concentrate sample, preferably add tert-butyl methyl ether or chloroform to prevent precipitation;
g) measure extract using LC-MS, with a High Resolution accurate Mass Spectrometer, preferably within 0 to 24 hours of extraction;
h) identify compounds present in the extract via accurate mass measurements and/or comparison to known standards;
i) compare concentration of identified compounds found in the solvent extracts of each of the garment portions, to enable a comparison between the sebum removal effects of each tested laundry detergent composition.

The method of the present invention is defined in claim 1.

In method step c), preferably at least one of the laundry detergent compositions comprises an ingredient selected from either alkyl ether carboxylate or lipid esterase.

Preferably the lipid esterase is selected from triacylglycerol lipase (E.C. 3.1.1.3); carboxylic ester hydrolase (E.C. 3.1.1.1); cutinase (E.C. 3.1.1.74); sterol esterase (E.C. 3.1.1.13); wax-ester hydrolase (E.C. 3.1.1.50), preferably the lipid esterase is a triacylglycerol lipase (E.C. 3.1.1.3).

Preferably the alkyl ether carboxylate is:

R-(OCH₂CH₂)ₙ-OCH₂COOH;

wherein R is an alkyl chain, linear or branched, preferably C₈ to C₂₄ and may be saturated or unsaturated;
and n is from 5 to 30, most preferably 9 to 20.

In method step c), the garment portions are preferably washed using 2 different laundry detergent compositions.

Preferably in method step b), the garment is portioned into 2 portions which are left/right mirror images of each other.

Preferably after method step a), the garment is stored for from 0 to 400 hours, preferably at a temperature below 313K, more preferably 253 to 303K, most preferably 283 to 298K. Preferably the solvent extraction of method step e) has a solvent to cloth weight ratio of 10:1 to 100:1, more preferably 20:1 to 40:1, and preferably the extraction is carried out for at least 1 hour.

It is preferable that the solvent extraction of method step e) is via Soxhlet extraction or supercritical CO₂ extraction.

Preferably the LC-MS of method step (g) is carried out using a High Resolution accurate Mass Spectrometer with electrospray ionisation and quadrupole time-of-flight mass spectrometry detection.

It is preferable to dilute extract by known amounts and remeasure to ensure spectrometer response is linear to the concentration of analyte.

For compounds of method step (h), it is preferable that fatty acids are analysed in negative mode and glycerides, wax esters, cholesterol esters and squalene in positive mode.

Preferably the laundry detergent composition used in this method comprises anionic surfactant, more preferably at levels of from 1 to 50 wt.%.

It is intended that any preferable subject matter described herein can be combined with any other subject matter, particularly combining 2 or more preferable subject matters.

### Detailed Description of the Invention

### Garments

Preferred garments are worn on the upper half of the body, and are preferably selected from T-Shirts, vests, shirts and blouses. Garments are preferable constructed from cellulosic fibres, polyester, acrylic, elastane and mixtures thereof, more preferably cotton, polyester and elastane. Cellulosic fibres include viscose, modal, and tencel.

Preferably garments are white.

Preferably garments are symmetrical so that the left and right half are mirror images of each other.

Preferably garments are preferably washed in a non-phosphate reference detergent products, such as SDCE Reference Detergent 4, before being worn or more preferably solvent extracted with preferably CO₂ to remove any organic matter from the surface of the garment. Non-phosphate reference detergence contain less than 1wt% of phoshorous containing ingredients.

### Washing method

The garments may be washed by any domestic wash habit, preferably by hand washing, washing in a top loading automatic washing machine or washing in a front loading automatic washing machine.

Preferably water hardnesses of 6 to 48 degrees French hard are used for washes and rinses, with liquor to cloth ratios in the range of 2:1 to 40:1, preferably 5:1 to 30:1.

Maximum wash water temperature is preferably 278 to 320K, more preferably 285 to 303K.

Rinse water temperatures are preferably ambient.

### Laundry detergent compositions

The laundry detergent compositions for testing may preferably be a powder detergent or a liquid detergent. The detergent may be in the form of a unit dose, for example a liquid unit dose.

Preferably two different laundry detergent compositions are compared.

### Soxhlet extraction

Soxhlet extraction is described in Harwood, L. M.; Moody, C. J. Experimental organic chemistry: Principles and Practice (Illustrated ed.). Wiley-Blackwell. 1989.

The solvents used in the solvent extraction are selected from alcohols, ketones, ethers, chlorinated organic solvent. Preferably the solvent is acetone or chloroform.

Supercritical CO₂ extraction is discussed in Taylor, Larry T (1996). Supercritical Fluid Extraction. Techniques in analytical chemistry. John Wiley and Sons, Inc.

### Chromatography

Ultra performance convergence, liquid chromatography (UPLC), and high performance liquid chromatography (HPLC) are preferred. Such systems are available fom Agilent, Waters, Thermo-fisher. HPLC method are described in Essentials in Modern HPLC Separations by Serban Moldoveanu and Victor David (Elsevier 2012).

### Mass Spectrometer

Mass Spectrometry is discussed in Mass Spectrometry Third Edition: Principles and Applications (Hoffmann E. Wiley-Interscience, 2007); Mass Spectrometry: A Textbook (Gross J.H. Springer 2017); Electrospray and MALDI Mass Spectrometry: Fundamentals, Instrumentation, Practicalities, and Biological Applications: Fundamentals, Instrumentation, and Applications (Cole, R.B. ed, Wiley Blackwell 2010).

Any High Resolution accurate mass spectrometer may be used.

High Resolution accurate mass spectrometry is mass spectrometry in which m/z for each ion is resolved and measured to several decimal places, preferably 4 decimal places. This is useful to differentiate between molecular formulas having the same nominal masses. Masses are measured in unified mass units, also referred to as Dalton.

Soft ionisation methods are preferred, preferably selected from laser desorption ionization (LDI), atmospheric pressure chemical ionization (APCI) and Electrospray ionisation (ESI), most preferably Electrospray ionisation (ESI).

LDI is preferably selected from matrix-assisted laser desorption ionization (MALDI) and surface assisted laser desorption ionization (SALDI). For MALDI the matrix is preferably selected from 2,5-dihydroxybenzoic acid and α-cyano-4-hydroxycinnamic acid (CHCA) with crystal sizes of 5 to 20 microns after spray deposition.

High resolution detectors are preferably selected from Fourier Transform Ion Cyclotron Resonance Mass Analyzers, Triple quadruple (QqQ) mass analyzers and Quadrupole-Time of Flights analyzers (Q-Tof). ESI with Q-ToF is most preferred.

In triple-quad instruments contain two quadrupoles for mass filtering flanking a third that acts as a collision cell. In MS mode, the quadrupole scans across an m/z range, sequentially transmitting each m/z value to produce an MS spectrum; in MS/MS mode it acts as a selective ion filter. In Quadrupole-Tof (Q-Tof) instruments, the quadrupole (and collision cell) is paired with a time-of-flight analyzer, allowing high-resolution, high mass accuracy analysis of all ions simultaneously.

The quadrupole mass filter consists of four parallel metal rods on which a fixed direct-current voltage and alternating radio-frequency voltage are applied, which allow selectively filtering of all ions except for those of a specified mass to charge ratio (m/z), depending on the applied voltage.

Mass Spectrometer may be purchased from providers such as: Sciex, Agilent, and Waters.

Examples of suitable spectrometers are:
Xevo G2-XS QTof Quadrupole Time-of-Flight Mass Spectrometry (Waters)
Vion IMS QTof Ion Mobility Quadrupole Time-of-flight Mass Spectrometry (Waters) SYNAPT G2-Si Mass Spectrometry (Waters)
6545 Q-TOF LC/MS (Agilent)

### Mass Spectral Analysis

The mass spectra acquired should be analysed for wax esters, cholesterol esters, triglycerides, diglycerides and monoglycerides, squalene and free fatty acids.

Sebum lipid analysis by mass spectrometry is described in Camera E., et al, Journal of lipid research volume 51, 2010, page 3377 to 3388 and reference therein.

Wax esters are of the form R₁-COO-R₂, where R₁ and R₂ are alkyl groups, preferably C11 to C24 linear alkyl chains which are saturated or mono-unsaturated.

Cholesterol esters are of the form: cholesterol-COO-R₃ where R₃ alkyl groups are preferably C12 to C24 linear alkyl chains which are saturated or mono-unsaturated.

Triglycerides are of the form 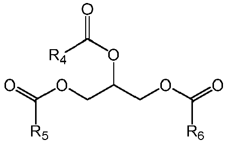 where R₄, R₅, and R₆ are alkyl groups, preferably independently selected from C9 to C23 linear alkyl chains which are saturated or mono-unsaturated.

Free fatty acids are of the from R₇-COOH where R₇ are alkyl groups preferably C12 to C24 linear alkyl chains which are saturated or mono-unsaturated.

Traces of other minor sebum and skin components may be present such as phospholipids, proteins, protein fragments, polyunsaturated lipids.

### Detergent formulations

The laundry detergent composition used in the method may be in any suitable form such as liquids, powders, bars, unit dose powders, unit dose liquids. Laundry detergent compositions may preferably contain surfactants such as linear alkyl benzene sulfonates, alkyl ether sulfates, fatty alcohol ethoxylates, alkyl sulfates, quaternary ammonium salts (rinse conditioner active). Preferably the laundry detergent composition comprises anionic surfactant, preferably at a level of from 1 to 50 wt.%.

Other common ingredient include: protease and amylase enzymes, sequesterants, builders, anti-redeposition polymers.

The inventive method is particularly good at showing the advantages of alkyl ether carboxylates and lipid esterases in detergent formulations with anionic surfactants.

### Alkyl Ether Carboxylates

Alkyl Ether Carboxylates are preferably of the form R-(OCH₂CH₂)ₙ-OCH₂COOH.

Preferably the R group of the alkyl ether carboxylate is an alkyl chain, linear or branched, preferably C₈ to C₂₄ and may be saturated or unsaturated;

Preferably n is from 5 to 30, most preferably 9 to 20.

They may be used as salt version for example sodium salt, or amine salt. Weights of alkyl ether carboxylic acid are calculated as the protonated form.

The alkyl chain, R is preferably linear.

The alkyl chain may be aliphatic or contain one cis or trans double bond.

The alkyl chain is most preferably selected from CH₃(CH₂)₁₁, CH₃(CH₂)₁₃, CH₃(CH₂)₁₅, CH₃(CH₂)₁₇, and CH₃(CH₂)₇CH=CH(CH₂)₈.

Alkyl ether carboxylic acid are available from Kao (Akypo ^{®}), Huntsman (Empicol^{®}) and Clariant (Emulsogen ^{®}). The sodium salt of the alkyl ether carboxylate is most preferred.

### Lipid Esterase

Cleaning lipid esterases are discussed in Enzymes in Detergency edited by Jan H. Van Ee, Onno Misset and Erik J. Baas (1997 Marcel Dekker, New York).

Cleaning lipid esterases are preferable active at alkaline pH in the range 7 to 11, most preferably they have maximum activity in the pH range 8 to 10.5.

The lipid esterase may be selected from lipase enzymes in E.C. class 3.1 or 3.2 or a combination thereof.

Preferably the cleaning lipid esterases is selected from:
(1) Triacylglycerol lipases (E.C. 3.1.1.3)
(2) Carboxylic ester hydrolase (E.C. 3.1.1.1)
(3) Cutinase (E.C. 3.1.1.74)
(4) Sterol esterase (E.C. 3.1.1.13)
(5) Wax-ester hydrolase (E.C. 3.1.1.50)

Suitable triacylglycerol lipases can be selected from variants of the Humicola lanuginosa (Thermomyces lanuginosus) lipase. Other suitable triacylglycerol lipases can be selected from variants of Pseudomonas lipases, e.g., from P. alcaligenes or P. pseudoalcaligenes (EP 218 272), P. cepacia (EP 331 376), P. stutzeri (GB 1,372,034), P. fluorescens, Pseudomonas sp. strain SD 705 (WO 95/06720 and WO 96/27002), P. wisconsinensis (WO 96/12012), Bacillus lipases, e.g., from B. subtilis (Dartois et al. (1993), Biochemica et Biophysica Acta, 1131, 253-360), B. stearothermophilus (JP 64/744992) or B. pumilus (WO 91/16422).

Suitable carboxylic ester hydrolases can be selected from wild-types or variants of carboxylic ester hydrolases endogenous to B. gladioli, P. fluorescens, P. putida, B. acidocaldarius, B. subtilis, B. stearothermophilus, Streptomyces chrysomallus, S. diastatochromogenes and Saccaromyces cerevisiae.

Suitable cutinases can be selected from wild-types or variants of cutinases endogenous to strains of Aspergillus, in particular Aspergillus oryzae, a strain of Alternaria, in particular Alternaria brassiciola, a strain of Fusarium, in particular Fusarium solani, Fusarium solani pisi, Fusarium oxysporum, Fusarium oxysporum cepa, Fusarium roseum culmorum, or Fusarium roseum sambucium, a strain of Helminthosporum, in particular Helminthosporum sativum, a strain of Humicola, in particular Humicola insolens, a strain of Pseudomonas, in particular Pseudomonas mendocina, or Pseudomonas putida, a strain of Rhizoctonia, in particular Rhizoctonia solani, a strain of Streptomyces, in particular Streptomyces scabies, a strain of Coprinopsis, in particular Coprinopsis cinerea, a strain of Thermobifida, in particular Thermobifida fusca, a strain of Magnaporthe, in particular Magnaporthe grisea, or a strain of Ulocladium, in particular Ulocladium consortiale.

In a preferred embodiment, the cutinase is selected from variants of the Pseudomonas mendocina cutinase described in WO 2003/076580 (Genencor), such as the variant with three substitutions at I178M, F180V, and S205G.

In another preferred embodiment, the cutinase is a wild-type or variant of the six cutinases endogenous to Coprinopsis cinerea described in H. Kontkanen et al, App. Environ. Microbiology, 2009, p2148-2157.

In another preferred embodiment, the cutinase is a wild-type or variant of the two cutinases endogenous to Trichoderma reesei described in WO2009007510 (VTT).

In a most preferred embodiment the cutinase is derived from a strain of Humicola insolens, in particular the strain Humicola insolens DSM 1800. Humicola insolens cutinase is described in WO 96/13580 which is hereby incorporated by reference. The cutinase may be a variant, such as one of the variants disclosed in WO 00/34450 and WO 01/92502. Preferred cutinase variants include variants listed in Example 2 of WO 01/92502. Preferred commercial cutinases include Novozym 51032 (available from Novozymes, Bagsvaerd, Denmark).

Suitable sterol esterases may be derived from a strain of Ophiostoma, for example Ophiostoma piceae, a strain of Pseudomonas, for example Pseudomonas aeruginosa, or a strain of Melanocarpus, for example Melanocarpus albomyces.

In a most preferred embodiment the sterol esterase is the Melanocarpus albomyces sterol esterase described in H. Kontkanen et al, Enzyme Microb Technol., 39, (2006), 265-273.

Suitable wax-ester hydrolases may be derived from Simmondsia chinensis.

The lipid esterase is preferably selected from lipase enzyme in E.C. class 3.1.1.1 or 3.1.1.3 or a combination thereof, most preferably E.C.3.1.1.3.

Examples of EC 3.1.1.3 lipases include those described in WIPO publications WO 00/60063, WO 99/42566, WO 02/062973, WO 97/04078, WO 97/04079 and US 5,869,438. Preferred lipases are produced by Absidia reflexa, Absidia corymbefera, Rhizmucor miehei, Rhizopus deleman Aspergillus niger, Aspergillus tubigensis, Fusaήum oxysporum, Fusarium heterosporum, Aspergillus oryzea, Penicilium camembertii, Aspergillus foetidus, Aspergillus niger, Thermomyces lanoginosus (synonym: Humicola lanuginosa) and Landerina penisapora, particularly Thermomyces lanoginosus. Certain preferred lipases are supplied by Novozymes under the tradenames. Lipolase^{®}, Lipolase Ultra^{®}, Lipoprime^{®}, Lipoclean^{®} and Lipex^{®} (registered tradenames of Novozymes) and LIPASE P "AMANO^{®}" available from Areario Pharmaceutical Co. Ltd., Nagoya, Japan, AMANO-CES^{®}, commercially available from Toyo Jozo Co., Tagata, Japan; and further Chromobacter viscosum lipases from Amersham Pharmacia Biotech., Piscataway, New Jersey, U.S.A. and Diosynth Co., Netherlands, and other lipases such as Pseudomonas gladioli. Additional useful lipases are described in WIPO publications WO 02062973, WO 2004/101759, WO 2004/101760 and WO 2004/101763. In one embodiment, suitable lipases include the "first cycle lipases" described in WO 00/60063 and U.S. Patent 6,939,702 B1, preferably a variant of SEQ ID No. 2, more preferably a variant of SEQ ID No. 2 having at least 90% homology to SEQ ID No. 2 comprising a substitution of an electrically neutral or negatively charged amino acid with R or K at any of positions 3, 224, 229, 231 and 233, with a most preferred variant comprising T23 IR and N233R mutations, such most preferred variant being sold under the tradename Lipex^{®} (Novozymes).

The aforementioned lipases can be used in combination (any mixture of lipases can be used). Suitable lipases can be purchased from Novozymes, Bagsvaerd, Denmark; Areario Pharmaceutical Co. Ltd., Nagoya, Japan; Toyo Jozo Co., Tagata, Japan; Amersham Pharmacia Biotech., Piscataway, New Jersey, U.S.A; Diosynth Co., Oss, Netherlands and/or made in accordance with the examples contained herein.

Lipid esterase with reduced potential for odor generation and a good relative performance, are particularly preferred, as described in WO 2007/087243. These include lipoclean ^{®} (Novozyme).

Following washing and drying, residual lipase activity on sebum on the garment may be curtailed by briefly heating to 80°C or immediate solvent extraction.

The invention will be further described with the following non-limiting examples.

### Examples

Unworn, new cotton or polycotton T-Shirts was worn by 22 volunteers for 1 day. Prior to wearing the T-shirts were washed in in a European front loading washing machine on a 40°C wash cycle with the control liquids formulation outlined below.

After wearing, the T-shirts were cut in half and one half washed in a control liquid formulation (16.7 wt.% total surfactant (LAS, SLES, Fatty alcohol ethoxylate and low level of soap as anti-foam) and one half washed in a matching formulation were the level of the original surfactant had been reduced to 9.4 wt.% and 4 wt.% of alkyl ether carboxylate added to giving 13.4 wt.% total surfactant.

The alkyl ether surfactant was the Na salt of cetearyl with 20 moles of ethoxylation.

All the T-shirt halves were place in a European front loading washing machine on a 40°C cycle with 35ml of the requisite product. After washing they were tumble dried, and the left and right halves rematched.

6g of fabric taken from the upper middle back area of the matching left and right halves of the shirts was removed, accurately weighed a placed in separate Soxhlet thimbles. The shirts were then Soxhlet extracted for 2 hours using 200ml of acetone at 95°C. After cooling, residual solvent in the cotton was removed by squeezing the liquid into the acetone containing the extract. The acetone was then removed using a rotary evaporator. Once all acetone was removed, 10ml of tert-butyl methyl ether and 10ml of Propan-2-ol is added to the extract. Vigorous shaking and swirling is applied to dissolve all material. The solvent is then decanted into a vial for analysis LC-MS analysis.

The LC-MS analysis was conducted on an Agilent 6530 Accurate-Mass Q-TOF LC/MS with following parameters

### HPLC analysis of Fatty Acids

| | |
|---|---|
| Column: | Agilent InfinityLab Poroshell 120 EC-C18 4.6 x 50mm, 2.7 micron |
| Flow rate: | 0.25ml/minute, Injection Volume 4 microlitres, Column Oven: 40°C |
| Eluents: | A = 5mM Ammonium Formate |
| | B = MeOH:Propan-2-ol 95:5 + 5mM Ammonium Formate |

### Timetable:

| | | | |
|---|---|---|---|
| 0 min | 30% A | 70% B | 0.25ml/min |
| 1 min | 30% A | 70% B | 0.25ml/min |
| 20 mins | 1% A | 99% B | 0.25ml/min |
| 32 mins | 1% A | 99% B | 0.25ml/min |
| 34 mins | 0% A | 100% B | 0.25ml/min |
| 50 mins | 30% A | 70% B | 0.25ml/min |
| 56 mins | 30% A | 70% B | 0.25ml/min |

### QTOF:

Negative polarity, m/z range: 80 - 3200
Gas Temp: 300°C, Gas Flow: 7 litres/min
Nebuliser: 50 psig, VCap: 4000V
Fragmentor: 200V, Skimmer: 65V

### HPLC analysis of neutral components

| | |
|---|---|
| Column: | ThermoScientific MOS-1 Hypersil 200mm x 2.1mm, 5 micron |
| Flow rate: | 0.25ml/minute, Injection Volume 4 microlitres, Column Oven: 50°C |
| Eluents: | A = 60:40 Water:Propan-2-ol + 25mM Ammonium Formate |
| | B = 10:10:80 Water:Propan-2-ol:n-butanol + 25mM Ammonium Formate |

### Timetable:

| | | | |
|---|---|---|---|
| 0 min | 70% A | 30% B | 0.25ml/min |
| 1.5 min | 70% A | 30% B | 0.25ml/min |
| 25 mins | 40% A | 60% B | 0.25ml/min |
| 28 mins | 0 % A | 100% B | 0.25ml/min |
| 34 mins | 70 % A | 30% B | 0.25ml/min |
| 60 mins | 70% A | 30% B | 0.25ml/min |

### QTOF:

Positive polarity, m/z range: 80 - 3200
Gas Temp: 300°C, Gas Flow: 7 litres/min
Nebuliser: 50 psig, VCap: 4000V, Fragmentor: 175V, Skimmer: 65V

The results are given in the table below (table 1) for the identified compounds.

For the triglyceride where more than 1 isomer is possible via substitution of the fatty acids on different OH groups of the glycerol, the value is given for the sum of all isomers.

The AEC formulation (shown in the table as AEC) removes more sebum than the control (without AEC shown in the table as Control), as evidenced by the lower signal size of the identified compounds in human sebum. The table shows the level of the compound found on the garment for both the control formulation and the AEC containing formulation, and the percentage level of the compound present in the AEC treated garment compared to the control treated garment. In all cases, the level of identified compound was lower in the AEC treated garment than the control.

This shows that the claimed method can show the effect of a laundry detergent composition on human sebum removal, and can demonstrate the sebum removal efficacy differences between different laundry detergent compositions.

**Table 1**

| **Compound** | **100*AEC/control** | **AEC peak integration** | **Control peak integration** |
|---|---|---|---|
| Myristic Acid | 75 | 1,294,591 | 1,719,369 |
| Palmitic Acid | 56 | 3,965,626 | 7,123,075 |
| Arachidic | 68 | 304,444 | 449,376 |
| Behenic | 67 | 1,447,352 | 2,160,009 |
| Sapienic | 89 | 3,667,927 | 4,133,466 |
| Oleic | 87 | 6,537,638 | 7,550,411 |
| Cholesterol Oleate | 74 | 3,101,352 | 4,198,801 |
| Isostearyl isostearate | 77 | 2,819,322 | 3,674,746 |
| Myristyl Myristate | 79 | 972,375 | 1,224,655 |
| Oleyl Oleate | 76 | 18,123,143 | 23,770,960 |
| Squalene | 78 | 3,057,619 | 3,904,885 |
| Palmitin | 61 | 1,087,199 | 1,772,990 |
| Glyceryl Tripalmitate | 71 | 11,313,315 | 15,950,621 |
| Glyceryl Trioleate | 76 | 3,289,289 | 4,301,676 |
| Glyceryl Tristearate | 78 | 2,941,432 | 3,779,704 |
| Glyceryl Distearate | 64 | 790,058 | 1,237,342 |
| Glyceryl Monostearate | - | 0 | 0 |
| Triglyceride: C16:1 C16:1 C16:1 | 75 | 2,089,504 | 2,783,019 |
| Triglyceride: C16:1 C16:1 C16:0 | 75 | 10,327,414 | 13,747,333 |
| Triglyceride: C16:1 C16:0 C16:0 | 74 | 16,953,258 | 22,827,044 |
| Wax ester: FA16:1-WE28:1 | 72 | 898,804 | 1,248,326 |
| Wax ester: FA16:1-WE30:1 | 69 | 5,736,416 | 8,327,112 |
| Wax ester: FA16:1-WE32:1 | 71 | 9,916,246 | 14,009,758 |
| Wax ester: FA16:1-WE34:2 | 77 | 6,423,426 | 8,307,812 |
| Wax ester: FA16:1-WE34:1 | 65 | 12,600,650 | 19,482,881 |
| Wax ester: FA16:1-WE36:1 | 72 | 13,994,327 | 19,364,542 |
| Wax ester: FA16:1-WE38:1 | 75 | 13,479,123 | 17,898,797 |

## Claims

1. A method of demonstrating sebum removal from laundered garment(s) by one or more laundry detergent compositions, said method comprising:
a) human subject to wear garment next to skin, preferably for a time of from 5 to 100 hours, more preferably the garment is worn on the upper half of the body;
b) the worn garment is portioned into 2 or more equal portions;
c) garment portions are washed separately to each other using different laundry detergent compositions to enable a comparison to be made;
d) The garment portions are dried and stored, preferably stored for a maximum of 96 hours at a temperature below 293K;
e) each garment portion or equal part thereof undergoes solvent extraction with solvent selected from: alcohols, ketones, ethers, CO₂, chlorinated organic solvent, preferably the solvent is acetone or chloroform;
f) take the solvent extract and choose method selected from:
(i) use as is; remove all solvent and then redissolve in a solvent mixture or
(ii) reduce solvent volume to concentrate sample, preferably add tert-butyl methyl ether or chloroform to prevent precipitation;
g) measure extract using LC-MS, with a High Resolution accurate Mass Spectrometer, preferably within 0 to 24 hours of extraction;
h) identify compounds present in the extract via accurate mass measurements and/or comparison to known standards;
i) compare concentration of identified compounds found in the solvent extracts of each of the garment portions, to enable a comparison between the sebum removal effects of each tested laundry detergent composition.

2. A method according to claim 1, wherein in method step c), at least one of the laundry detergent compositions comprises an ingredient selected from either alkyl ether carboxylate and/or lipid esterase.

3. A method according to claim 2 wherein the lipid esterase is selected from triacylglycerol lipase (E.C. 3.1.1.3); carboxylic ester hydrolase (E.C. 3.1.1.1); cutinase (E.C. 3.1.1.74); sterol esterase (E.C. 3.1.1.13); wax-ester hydrolase (E.C. 3.1.1.50), preferably the lipid esterase is a triacylglycerol lipase (E.C. 3.1.1.3).

4. A method according to claim 2 wherein the alkyl ether carboxylate is:
R-(OCH₂CH₂)ₙ-OCH₂COOH;
wherein R is an alkyl chain, linear or branched,
and n is from 5 to 30, most preferably 9 to 20.

5. A method according to any preceding claim, wherein in method step c), the garment portions are washed using 2 different laundry detergent compositions.

6. A method according to any preceding claim, wherein in method step b), the garment is portioned into 2 portions which are left/right mirror images of each other.

7. A method according to any preceding claim, wherein after method step a), the garment is stored for from 0 to 400 hours, preferably at a temperature below 313K, more preferably 253 to 303K, most preferably 283 to 298K.

8. A method according to any preceding claim, wherein the solvent extraction of method step e) has a solvent to cloth weight ratio of 10:1 to 100:1, preferably 20:1 to 40:1 and preferably the extraction is carried out for at least 1 hour.

9. A method according to any preceding claim, wherein the solvent extraction of method step e) is via Soxhlet extraction or supercritical CO₂ extraction.

10. A method according to any preceding claim, wherein the LC-MS of method step (g) is carried out using a High Resolution accurate Mass Spectrometer with electrospray ionisation and quadrupole time-of-flight mass spectrometry detection.

11. A method according to any preceding claim, wherein for compounds of method step (h), fatty acids are analysed in negative mode and glycerides, wax esters, cholesterol esters and squalene in positive mode.

12. A method according to any preceding claim, wherein the laundry detergent composition comprises anionic surfactant, preferably at levels of from 1 to 50 wt.%

## Revendications

1. Procédé de démonstration d'élimination de sébum de vêtement(s) lavé(s) par une ou plusieurs compositions de détergent de lavage, ledit procédé comprenant :
a) un humain sujet à porter un vêtement à proximité de la peau, de préférence sur une durée de 5 à 100 heures, encore mieux le vêtement est porté sur la moitié supérieure du corps ;
b) le vêtement porté est divisé en 2 portions égales ou plus ;
c) les portions de vêtement sont lavées séparément les unes des autres en utilisant différentes compositions de détergent de lavage pour permettre de faire une comparaison ;
d) les portions de vêtement sont séchées et stockées, de préférence stockées pendant un maximum de 96 heures à une température inférieure à 293 K ;
e) chaque portion de vêtement ou partie égale de celle-ci est soumise à une extraction au solvant avec un solvant choisi parmi : des alcools, cétones, éthers, COz, solvant organique chloré, le solvant est de préférence l'acétone ou le chloroforme ;
f) le prélèvement de l'extrait de solvant et le choix d'un procédé choisi parmi :
(i) l'utilisation tel qu'il est ; l'élimination de la totalité du solvant puis la redissolution dans un mélange de solvant, ou
(ii) la réduction du volume de solvant pour concentrer l'échantillon, de préférence l'addition de tert-butyl méthyl éther ou de chloroforme pour éviter une précipitation ;
g) la mesure de l'extrait en utilisant LC-MS, avec un spectromètre de masse précis haute résolution, de préférence en de 0 à 24 heures d'extraction ;
h) l'identification des composés présents dans l'extrait via des mesures de masse précises et/ou la comparaison aux normes connues ;
i) la comparaison d'une concentration de composés identifiés trouvés dans les extraits de solvant de chacune des portions de vêtement, pour permettre une comparaison entre les effets d'élimination du sébum de chaque composition de détergent de lavage testée.

2. Procédé selon la revendication 1, dans lequel dans l'étape c) du procédé, au moins une des compositions de détergent de lavage comprend un ingrédient choisi parmi soit un carboxylate d'alkyl éther et/soit une lipide estérase.

3. Procédé selon la revendication 2, dans lequel la lipide estérase est choisie parmi une triacylglycérol lipase (E.C. 3.1.1.3) ; hydrolase d'ester carboxylique (E.C. 3.1.1.1.) ; cutinase (E.C. 3.1.1.74) ; stérol estérase (E.C. 3.1.1.13) ; cire-ester hydrolase (E.C. 3.1.1.50), la lipide estérase est de préférence une triacylglycérol lipase (E.C. 3.1.1.3).

4. Procédé selon la revendication 2, dans lequel le carboxylate d'alkyl éther est :
R-(OCH₂CH₂)ₙ-OCH₂COOH ;
où R est un chaîne alkyle, linéaire ou ramifiée, et n est de 5 à 30, encore mieux de 9 à 20.

5. Procédé selon l'une quelconque des revendications précédentes, dans lequel dans l'étape c) du procédé, les portions de vêtement sont lavées en utilisant 2 compositions de détergent de lavage différentes.

6. Procédé selon l'une quelconque des revendications précédentes, où dans l'étape b) du procédé, le vêtement est divisé en 2 portions qui sont des images gauche/droite en miroir l'une de l'autre.

7. Procédé selon l'une quelconque des revendications précédentes, où après l'étape a) du procédé, le vêtement est stocké pendant de 0 à 400 heures, de préférence à une température inférieure à 313 K, encore mieux de 253 à 303 K, bien mieux encore de 283 à 298 K.

8. Procédé selon l'une quelconque des revendications précédentes, dans lequel l'extraction au solvant de l'étape e) du procédé présente un rapport de masse solvant à tissu de 10:1 à 100:1, de préférence de 20:1 à 40:1 et l'extraction est de préférence réalisée pendant au moins 1 heure.

9. Procédé selon l'une quelconque des revendications précédentes, dans lequel l'extraction au solvant de l'étape e) du procédé se fait via extraction Soxhlet ou extraction au COz supercritique.

10. Procédé selon l'une quelconque des revendications précédentes, dans lequel le LC-MS de l'étape g) du procédé est réalisé en utilisant un spectromètre de masse précis haute résolution avec une ionisation par électropulvérisation et une détection de spectrométrie de masse à temps de vol quadrupolaire.

11. Procédé selon l'une quelconque des revendications précédentes, dans lequel pour les composés de l'étape h) du procédé, des acides gras sont analysés en mode négatif et des glycérides, esters de cire, esters de cholestérol et squalène en mode positif.

12. Procédé selon l'une quelconque des revendications précédentes, dans lequel la composition de détergent de lavage comprend un tensioactif anionique, de préférence à des teneurs de 1 à 50 % en masse.

## Patentansprüche

1. Verfahren zur Demonstration der Sebumentfernung aus gewaschenen Kleidungsstücken durch ein oder mehrere Waschmittelzusammensetzung(en), wobei das Verfahren umfasst:
a) ein menschliches Subjekt zum Tragen eines Kleidungsstücks direkt auf der Haut, vorzugsweise für eine Zeit von 5 bis 100 Stunden, bevorzugter wird das Kleidungsstück auf der oberen Hälfte des Körpers getragen;
b) das getragene Kleidungsstück wird in 2 oder mehr gleiche Teile aufgeteilt;
c) die Kleidungsstückteile werden getrennt voneinander unter Verwendung verschiedener Waschmittelzusammensetzungen gewaschen, um einen Vergleich zu ermöglichen;
d) die Kleidungsstückteile werden getrocknet und gelagert, vorzugsweise maximal 96 Stunden bei einer Temperatur unter 293 K;
e) jedes Kleidungsstückteil oder ein gleicher Teil davon wird einer Lösungsmittelextraktion mit einem Lösungsmittel unterzogen, das aus Alkoholen, Ketonen, Ethern, CO₂, chloriertem organischem Lösungsmittel ausgewählt ist, wobei das Lösungsmittel vorzugsweise Aceton oder Chloroform ist;
f) nimm den Lösungsmittelextrakt und wähle eine Methode, ausgewählt aus:
(i) verwende ihn, wie er ist; entferne das gesamte Lösungsmittel und löse dann erneut in einem Lösungsmittelgemisch; oder
(ii) reduziere das Lösungsmittelvolumen, um die Probe zu konzentrieren, gib vorzugsweise tert-Butylmethylether oder Chloroform hinzu, um eine Ausfällung zu verhindern;
g) messe den Extrakt unter Verwendung von LC-MS mit einem hochauflösenden, präzisen Massenspektrometer, vorzugsweise innerhalb von 0 bis 24 Stunden nach der Extraktion;
h) identifiziere die in dem Extrakt vorliegenden Verbindungen durch präzise Massenmessungen und/oder Vergleich mit bekannten Standards;
i) vergleiche die Konzentration der identifizierten Verbindungen, die in den Lösungsmittelextrakten von jedem der Kleidungsstückteile gefunden wurden, um einen Vergleich zwischen den Sebumentfernungseffekten jeder getesteten Waschmittelzusammensetzung zu ermöglichen.

2. Verfahren nach Anspruch 1, wobei im Verfahrensschritt c) mindestens eine der Waschmittelzusammensetzungen einen Bestandteil umfasst, ausgewählt aus entweder Alkylethercarboxylat und/oder Lipidesterase.

3. Verfahren nach Anspruch 2, wobei die Lipidesterase aus Triacylglycerollipase (E.C. 3.1.1.3); Carbonsäureesterhydrolase (E.C. 3.1.1.1); Cutinase (E.C. 3.1.1.74); Sterolesterase (E.C. 3.1.1.13); Wachsesterhydrolase (E.C. 3.1.1.50) ausgewählt ist, wobei die Lipidesterase vorzugsweise eine Triacylglycerollipase (E.C. 3.1.1.3) ist.

4. Verfahren nach Anspruch 2, wobei das Alkylethercarboxylat
R-(OCH₂CH₂)ₙ-OCH₂COOH
ist,
wobei R eine Alkylkette, linear oder verzweigt, ist,
und wobei n 5 bis 30, höchst bevorzugt 9 bis 20, beträgt.

5. Verfahren nach irgendeinem vorhergehenden Anspruch, wobei im Verfahrensschritt c) die Kleidungsstückteile unter Verwendung von 2 unterschiedlichen Waschmittelzusammensetzungen gewaschen werden.

6. Verfahren nach einem vorhergehenden Anspruch, wobei im Verfahrensschritt b) das Kleidungsstück in 2 Teile aufgeteilt wird, die linke/rechte Spiegelbilder voneinander sind.

7. Verfahren nach einem vorhergehenden Anspruch, wobei nach dem Verfahrensschritt a) das Kleidungsstück 0 bis 400 Stunden, vorzugsweise bei einer Temperatur unter 313 K, bevorzugter bei 253 bis 303 K, höchst bevorzugt bei 283 bis 298 K, gelagert wird.

8. Verfahren nach einem vorhergehenden Anspruch, wobei die Lösungsmittelextraktion des Verfahrensschritts e) ein Gewichtsverhältnis von Lösungsmittel zu Stoff von 10:1 bis 100:1, vorzugsweise von 20:1 bis 40:1 aufweist und wobei die Extraktion vorzugsweise mindestens 1 Stunde durchgeführt wird.

9. Verfahren nach einem vorhergehenden Anspruch, wobei die Lösungsmittelextraktion des Verfahrensschritts e) über eine Soxhlet-Extraktion oder überkritische CO₂-Extraktion durchgeführt wird.

10. Verfahren nach einem vorhergehenden Anspruch, wobei die LC-MS des Verfahrensschritts (g) unter Verwendung eines hochauflösenden, präzisen Massenspektrometers mit Elektrospray-Ionisation und Quadrupol-Flugzeit-Massenspektrometrie-Detektion durchgeführt wird.

11. Verfahren nach einem vorhergehenden Anspruch, wobei für Verbindungen des Verfahrensschritts (h) Fettsäuren im negativen Modus und Glyceride, Wachsester, Cholesterinester und Squalen im positiven Modus analysiert werden.

12. Verfahren nach einem vorhergehenden Anspruch, wobei die Waschmittelzusammensetzung anionisches Tensid, vorzugsweise in Mengen von 1 bis 50 Gew.-%, umfasst.
